(19) Europäisches Patentamt
European Patent Office
Office européen des brevets

(11) **EP 2 108 357 B1**

(12) **EUROPÄISCHE PATENTSCHRIFT**

(45) Veröffentlichungstag und Bekanntmachung des Hinweises auf die Patenterteilung:
**12.02.2014 Patentblatt 2014/07**

(51) Int Cl.:
*A61K 6/083* [(2006.01)]

(21) Anmeldenummer: 08007196.2

(22) Anmeldetag: **11.04.2008**

(54) **Konditioniermittel für das Ätzen von Schmelzläsionen**

Conditioning agent for the etching of enamel lesions

Produit de conditionnement pour graver les lésions de l'émail dentaire

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**14.10.2009 Patentblatt 2009/42**

(73) Patentinhaber:
• **Mühlbauer Technology GmbH**
  **22547 Hamburg (DE)**
• **Charité - Universitätsmedizin Berlin**
  **10117 Berlin (DE)**

(72) Erfinder:
• **Neffgen, Stephan**
  **22459 Hamburg (DE)**
• **Neander, Swen Dr.**
  **20148 Hamburg (DE)**
• **Lübbers, Dierk Dr.**
  **25469 Halstenbek (DE)**

(74) Vertreter: **Glawe, Delfs, Moll**
**Partnerschaft mbB von**
**Patent- und Rechtsanwälten**
**Rothenbaumchaussee 58**
**20148 Hamburg (DE)**

(56) Entgegenhaltungen:
WO-A-00/09030    WO-A-2007/131725
US-A- 4 348 381    US-A- 6 036 944

• **MEYER-LUECKEL H ET AL: "SURFACE LAYER EROSION OF NATURAL CARIES LESIONS WITH PHOSPHORIC AND HYDROCHLORIC ACID GELS IN PREPARATION FOR RESIN INFILTRATION" CARIES RESEARCH, S. KARGER AG, BASEL, CH, Bd. 41, Nr. 3, 1. April 2007 (2007-04-01), Seiten 223-230, XP008078691 ISSN: 0008-6568**
• **MEYER-LUECKEL ET AL: "Influence of the application time on the penetration of different dental adhesives and a fissure sealant into artificial subsurface lesions in bovine enamel" DENTAL MATERIALS, ELSEVIER, Bd. 22, Nr. 1, 1. Januar 2006 (2006-01-01), Seiten 22-28, XP005214018 ISSN: 0109-5641**

**Beschreibung**

Die Erfindung betrifft eine Zusammensetzung zur Verwendung als Konditioniermittel für das Ätzen von Schmelzläsionen sowie ein Kit zur Infiltration von Schmelzläsionen.

Karies ist eine weitverbreitete Zivilisationskrankheit. Jede letztendlich zu einer Kavitation führende Karieserkrankung beginnt mit einer Demineralisierung des Zahnschmelzes und wird in diesem Stadium initiale Zahnschmelzkaries genannt. In diesem Stadium ist die Karies zunächst nicht oder lediglich als sogenannte weiße Zahnschmelzläsion sichtbar, führt aber zu einem porösen Bereich des Zahns unterhalb dessen Oberfläche.

Eine solche Schmelzläsion kann grundsätzlich remineralisiert oder mit einem Kunstharz infiltriert werden (WO 2007/131725 A1). Problematisch bei der Infiltration mit Kunstharz ist allerdings, dass typischerweise eine in den Zahnschmelz hineinreichende Läsion an der Zahnoberfläche eine sogenannte pseudointakte Oberflächenschicht aufweist, die einen höheren Mineralgehalt im Vergleich zu der tiefergehenden Schmelzläsion aufweist und die Penetrierung der Schmelzläsion mit dem zur Infiltration verwendeten Kunstharz, dem sogenannten Infiltranten, erschwert oder verhindert. Es ist daher bereits vorgeschlagen worden, diese pseudointakten Oberflächenschichten durch ein Ätzmittel vorzubehandeln, so dass nach diesem Konditionieren der Infiltrant besser in die Läsion einpenetrieren kann.

Der Erfindung liegt die Aufgabe zugrunde, ein wirksames und gut lagerfähiges und anwendbares Konditioniermittel für das Ätzen von Schmelzläsionen zu schaffen.

Die Erfindung betrifft somit eine Zusammensetzung, die enthält:

- 1 bis 80 Gew.-% wenigstens einer Säure, die einen pKs-Wert von 2 oder weniger aufweist;

- ein protisches Lösungsmittel;

- einen Komplexbildner für $Ca^{2+}$ - Ionen;
  zur Verwendung als Konditioniermittel für das Ätzen von Schmelzläsionen.

Die Säure mit einem pKs-Wert von 2 oder weniger kann eine anorganische oder organische Säure sein, Mischungen mehrerer Säuren sind ebenfalls möglich. Soweit es sich um eine mehrbasige Säure handelt, muss die erste Dissoziationsstufe aus dem in der Zusammensetzung vorliegenden Zustand der Säure einen pKs-Wert von 2 oder weniger aufweisen.

Bei dem protischen Lösungsmittel kann es sich insbesondere um Alkohole oder Wasser bzw. Mischungen daraus handeln.

Bei dem Komplexbildner für $Ca^{2+}$ Ionen kann es sich um jeden Komplexbildner handeln, der unter den Bedingungen der Anwendung der Zusammensetzung im Mundmilieu herausgelöste $Ca^{2+}$ Ionen komplexiert, vorzugsweise weitgehend vollständig oder zu einem erheblichen Teil komplexiert.

Die Erfindung hat erkannt, dass im Stand der Technik (WO 2007/131725 A1) als Konditioniermittel verwendete Salzsäure eine Reihe von Nachteilen aufweist. Um als Konditioniermittel hinreichend wirksam zu sein, muss eine etwa 15%ige Salzsäure verwendet werden. HCl besitzt in höher konzentrierten wässrigen Lösungen einen erheblichen Dampfdruck, so dass es gasförmig aus der wässrigen Salzsäurelösung entweicht. Entsprechende Salzsäurelösungen sind also wenig lagerstabil; zudem bestehen wegen der hohen Aggressivität des gasförmig entweichenden HCl hohe Anforderungen an die Beständigkeit der Verpackung des Konditioniermittels. Ferner kann bei der Anwendung entweichendes gasförmiges HCl biologische Gewebe wie beispielsweise das Zahnfleisch schädigen.

Die Erfindung hat erkannt, dass die im Patentanspruch näher definierte Kombination aus Säuren mit einem bestimmten Mindestwert der Säurestärke und Komplexbildner die pseudointakte Oberflächenschichten natürlicher Schmelzläsionen wirksam erodieren und damit für das Einpenetrieren eines Infiltranten vorbereiten können. Solche pseudointakte Oberflächenschichten einer natürlichen Schmelzläsion sind typischerweise 10 bis 40 μm dick und werden durch eine erfindungsgemäße Zusammensetzung dennoch in handhabbaren Applikationszeiten (beispielsweise etwa 90 bis 120 s) weitgehend vollständig durchdrungen und entfernt.

Überaschenderweise kann die Kombination einer Säure mit einem eigentlich eher passiven $Ca^{2+}$ Ionenkomplexbildner die beschriebenen remineralisierten Oberflächenschichten hinreichend ätzen. Dies war nicht zu erwarten, da Komplexbildung durch starke Säuren eigentlich unterdrückt wird. Zudem kann erfindungsgemäß überraschenderweise die zur Erzielung einer hinreichenden Ätzwirkung erforderliche Konzentration der Säure deutlich reduziert werden. Bei der Verwendung flüchtiger Säuren wie beispielsweise Salzsäure führt dies dazu, dass es bei der Lagerung und Anwendung der Konditioniermittel nicht oder in einem nur geringem Umfang zu einem Ausgasen aggressiver Säurebestandteile kommt.

Bevorzugt werden sehr starke Säuren mit einem pKs-Wert von 1 oder kleiner, weiter vorzugsweise 0 oder kleiner verwendet. Beispielsweise kann der pKs-Wert der verwendeten Säure zwischen -10 und 1, weiter vorzugsweise zwischen -10 und 0 betragen.

Die Säure kann erfindungsgemäß ausgewählt sein aus der Gruppe bestehend aus Perchlorsäure, Salzsäure, Schwefelsäure, Salpetersäure, Fluorsulfonsäure, organische Sulfonsäuren wie bspw. Trifluormethansulfonsäure, Methansulfonsäure, Toluolsulfonsäure, Amidosulfonsäure, Benzolsulfonsäure, Trichloressigsäure, Trifluoressigsäure, Pikrinsäure und Semiquadratsäure; weiter bevorzugt ausgewählt aus der Gruppe bestehend aus Salzsäure, Salpetersäure, Methansulfonsäure und Toluolsulfonsäure. Der Säuregehalt kann erfindungsgemäß vorzugsweise zwischen 1 und 50 Gew.-%, weiter vorzugsweise 4 und 30 Gew.-%, weiter vorzugsweise 4 und 15 Gew.-% liegen.

Erfindungsgemäß wird bevorzugt ein mehrzähniger Komplexbildner, vorzugsweise ein Chelatbildner verwendet. Er kann erfindungsgemäß ausgewählt sein aus der Gruppe bestehend aus Ethylendiamintetraessigsäure (EDTA), Diethylentriaminpentaessigsäure (DTPA), Nitrilotriessigsäure (NTA), Iminodiessigsäure (IDA), Glycolsäure, Citronensäure, 4-Hydroxybutansäure, Ethylendiamintetra(methylenphosphonsäure) (DTPMP), Diethylentriaminpenta(methylenphosphonsäure) (EDTMP), Aminotrismethylenphosphonsäure (ATMP), Hydroxyethylaminodi(methylenphosphonsäure) (HEMPA), Hexamethylendiamintetra(methylenphosphonsäure) (HDTMP), Phosphonobutan-1,2,4-tricarbonsäure (PBTC), 1-Hydroxyethan-1,1-diphosphonsäure (HEDP), Polyacrylsäure und deren Copolymere und Polyvinylphosphonsäure. Eine bevorzugte Untergrenze für den Gehalt an Komplexbildnern der erfindungsgemäßen Zusammensetzung ist 0,1 Gew.-%, weitere bevorzugte Untergrenzen sind 1, 5 und 10 Gew.-%. Der Komplexbildner kann in der Zusammensetzung bis zur Sättigungsgrenze enthalten sein, bevorzugte Obergrenzen für den Gehalt an Komplexbildner sind 80 Gew.-%, 60 Gew.-% und 40 Gew.-%. Die genannten Ober- und Untergrenzen lassen sich beliebig zu erfindungsgemäßen Bereichen kombinieren.

Die erfindungsgemäß eingesetzten Komplexbildner weisen bevorzugt eine hohe Affinität zu divalenten Kationen, insbesondere $Ca^{2+}$ Ionen auf. Bevorzugt ist eine gute Verträglichkeit mit dem Gewebe des Mundraums, insbesondere Zahnfleisch.

Komplexbildner oder insbesondere Chelatbildner können erfindungsgemäß auch in Salzform eingesetzt werden. Bevorzugt sind Salze monovalenter Kationen, beispielsweise das Dinatriumsalz des ETDA.

Die erfindungsgemäße Zusammensetzung kann zusätzlich Verdickungsmittel wie beispielsweise partikuläre Füllstoffe oder höher viskose Stoffe enthalten. Geeignete Verdickungsmittel sind beispielsweise Kieselsäuren, Polyether oder Polyole sowie Polyacrylsäuren und deren Copolymere. Weitere Zusatzstoffe wie beispielsweise Farbstoffe oder grenzflächenaktive Stoffe (Tenside) können ebenfalls enthalten sein.

Gegenstand der Erfindung ist ferner ein Kit zur Durchführung einer Infiltration von Zahnschmelz, der als Bestandteile eine erfindungsgemäße Konditioniererzusammensetzung sowie einen Infiltranten aufweist. Bei dem Infiltranten handelt es sich bevorzugt um ein härtbares (beispielsweise lichthärtbares) Kunstharz, das nach der Vorbereitung der Läsion durch den Konditionierer in diese Läsion einpenetrieren und diese versiegeln kann.

Bevorzugt weist in diesem Kit der verwendete Infiltrant einen Penetrationskoeffizienten von mehr als 50 cm/s auf. Der Penetrationskoeffizient errechnet sich nach der nachfolgend aufgeführten, aus der sogenannten Washburn-Gleichung ableitbaren Gleichung:

$$PC = \left( \frac{\gamma \cdot \cos\theta}{2\eta} \right)$$

PC: Penetrationskoeffizient
$\gamma$: Oberflächenspannung des Infiltranten an der Grenzfläche zur Luft
$\theta$: Kontaktwinkel des Infiltranten an der Grenzfläche zur Schmelz
$\eta$: dynamische Viskosität des Infiltranten

Weitere Angaben zur Bestimmung des Penetrationskoeffizienten einschließlich Literaturhinweisen finden sich in der bereits genannten WO 2007/131725 A1, die durch Bezugnahme auch zum Gegenstand der vorliegenden Offenbarung gemacht wird.

Bevorzugt enthält der Infiltrant wenigstens ein Harz ausgewählt aus der Gruppe bestehend aus MMA, Methylmethacrylat; EMA, Ethylmethacrylat; n-BMA, n-Butylmethacrylat; IBMA, Isobutylmethacrylat, t-BMA, tert-Butylmethacrylat; EHMA, 2-Ethylhexylmethacrylat; LMA, Laurylmethacrylat; TDMA, Tridecylmethacrylat; SMA, Stearylmethacrylat; CHMA, Cyclohexylmethacrylat; BZMA, Benzylmethacrylat; IBXMA, Isobornylmethacrylate; MAA, Methacrylsäure; HEMA, 2-Hydroxyethylmethacrylat; HPMA, 2-Hydroxypropylmethacrylat; DMMA, Dimethylaminoethylmethacrylat; DEMA, Diethylaminoethylmethacrylat; GMA, Glycidylmethacrylat; THFMA, Tetrahydrofurfurylmethacrylat; AMA, Allylmethacrylat; EGDMA, Ethylenglycoldimethacrylat; 3EGDMA, Triethylenglycoldimethacrylat; 4EGDMA, Tetraethylenglycoldimethacrylat; BDMA, 1,3-Butylenglycoldimethacrylat; HDDMA, 1,6-Hexandioldimethacrylat; ETMA, Ethoxyethylmethacrylat;

3FM, Trifluorethylmethacrylat; 8FM, Octafluorpentylmethacrylat; AIB, Isobutylacrylat; TBA, tert-Butylacrylat; LA, Laurylacrylat; CEA, Cetylacrylat; STA, Stearylacrylat; CHA, Cyclohexylacrylat; BZA, Benzylacrylat; IBXA, Isobornylacrylat; 2-MTA, 2-Methoxyethylacrylat; ETA, 2-Ethoxyethylacrylat; EETA, Ethoxyethoxyethylacrylat; PEA, 2-Phenoxyethylacrylat; THFA, Tetrahydrofurfurylacrylat; HEA, 2-Hydroxyethylacrylat; HPA, 2-Hydroxypropylacrylat; 4HBA, 4-Hydroxybutylacrylat; DMA, Dimethylaminoethylacrylat; 1,4-Butylendioldiacrylat; 4EDA, Tetraethylenglycoldiacrylat; NDDA, 1,9-Nonandioldiacrylat; 3F, Trifluorethylacrylat; 17F, Heptadecafluorodecylacrylat; 2-PEA, 2-Phenoxyethylacrylat; TBCH, 4-tert-butylcyclohexylacrylat; DCPA, Dihydrodicyclopentadienylacrylat; EHA, 2-Ethylhexylacrylat; 3EGMA, Triethylenglycolmonomethacrylat; DEGDMA, Diethylenglycoldimethacrylat; PDDMA, 1,5-Pentandioldimethacrylat; BDDMA, 1,4-Butandioldimethacrylat; PRDMA, 1,3-Propandioldimethacrylat (Viskosität < 30 mPas) oder aus der Gruppe bestehend aus DDDMA, 1,10-Decandioldimethacrylat; PEG400DA, Polyethylenglycol 400 Diacrylat, TMPTMA, Trimethylolpropantrimethacrylat, TMPTA, Trimethylolpropantriacrylat; DTMPTA; Di-Trimethylolpropantetraacrylat; DiPENTA, Di-Pentaerythritolpentaacrylat; PEG400DMA, Polyethylenglycol 400 Dimethacrylat, PEG300DA, Polyethylenglycol 300 Diacrylat, PEG300DMA, Polyethylenglycol 300 Dimethacrylat, BPA(EO)10DMA, Ethoxyliertes (10) Bisphenol-A-Dimethacrylat; BPA(EO)30DMA, Ethoxyliertes (30) Bisphenol-A-Dimethacrylat; PEG200DA, Polyethylenglycol 200 Diacrylat, PEG600DA, Polyethylenglycol 600 Diacrylat; NPG(PO)2DA Propoxyliertes (2) Neopentylglycol Diacrylat; BPA(EO)2DA, Ethoxyliertes (4) Bisphenol-A-Diacrylat; GPTA; propoxyliertes Glyceryltriacrylat; DMTCDDA, Dimethylol tricyclo [5.2.1.0$^{2,6}$]decandimethacrylat; BPA(PO)2DMA, Propoxyliertes (2) Bisphenol-A-Dimethacrylat; DPEHA, Dipentaerythritolhexaacrylat; Bis-GMA, 2,2-bis[4-(2-Hydroxy-3-Methacryloxypropoxy)phenyl]propan; und UDMA, 1,6-bis(Methacryloxy-2-Ethoxycarbonylamino)-2,4,4-trimethylhexan; (alle vorstehenden Harze haben eine Viskosität > 30 mPas).

Weitere bevorzugte Untergruppen für die Auswahl des Infiltranten sind Triethylenglycoldimethacrylat und Trimethylolpropantrimethacrylat.

Der erfindungsgemäße Kit kann Applikationsstreifen, Reinigungsstreifen und/oder Trenneinrichtungen enthalten. Bei der Trenneinrichtung handelt es sich um ein Instrument, mit dem sich Approximalflächen zweier benachbarter Zähne geringfügig (beispielsweise ca. 300 μm) beabstanden lassen. Reinigungsstreifen können mit einer entsprechenden Reinigungslösung zur vorbereitenden Reinigung zu behandelnder Dentalflächen versehen sein. Applikationsstreifen können mit dem Konditioniermittel bzw. dem Infiltranten getränkt sein und so ein einfaches und präzises Applizieren dieser Bestandteile des Kits auch auf schwer zugängliche Approximalflächen von Zähnen ermöglichen.

In den nachfolgenden Ausführungsbeispielen und Vergleichsbeispielen wird die Eignung verschiedener Konditioniermittel zum Erodieren bzw. Demineralisieren von remineralisierten Oberflächenschichten natürlicher Schmelzläsionen an einem Modellsystem geprüft.

Dabei wurde die Ätzwirkung auf Hydroxylapatit [Ca$_5$(PO$_4$)$_3$OH] (Hydroxylapatit-Plättchen) als Modellsystem für Schmelz, der zu etwa 95 % aus Hydroxylapatit besteht, untersucht. Unterschiedliche Konditioniermittel- oder Konditionierlösungen können hiermit unter reproduzierbaren Bedingungen bzgl. ihrer Ätzwirkung eingeschätzt und klassifiziert werden. Das Verfahren wird in der Publikation von A. Klocke et al., Dental Materials 19 (2003), 773 beschrieben. Die Hydroxylapatit-Plättchen wurden durch hydraulisches Pressen (Einwaage: 300mg; Druck: 5000 bar; Zeit: 35min) von getrocknetem (2h, 60°C) Hydroxylapatit (Riedel de Haeu) mit dem IR-Presswerkzeug (Firma LOT GmbH) hergestellt. Anschließend wurden die Presslinge so in einen Kunststoff (Paladur®, Heraeus Kulzer GmbH) eingebettet, dass nur eine Fläche unbedeckt blieb. Diese Fläche wurde mit Schleifpapier (Körnung: 500 und 1200) bearbeitet, um möglichst reproduzierbare Oberflächen zu erhalten und mit Pressluft vom Schleifstaub befreit. Der Durchmesser der Hydroxylapatitfläche betrug 13 mm.

Für die Ätzversuche wurden die Hydroxylapatit-Plättchen in 4 ml Konditionierlösung getaucht und im verschlossenen Probegefäß für 2 min auf einem Schüttelapparat (GFL; 100 min$^{-1}$) gerüttelt. Anschließend wurden die Hydroxylapatit-Plättchen vorsichtig mit einer Pinzette aus der Lösung genommen und mit Wasser (Qualität: Hyperpur) gründlich abgespült. Das Spülwasser wurde zurück in das Probegefäß gegeben. Der Inhalt des Probengefäßes wurde anschließend in einen 100 ml Meßkolben überführt, wobei das Probegefäß noch 2 mal mit Wasser ausgespült und das Waschwasser ebenfalls im Meßkolben aufgefangen wurde. Der Meßkolben wurde stets bis zur 100 ml-Eichmarke mit Wasser aufgefüllt. Mittels Atomabsorptionsspektroskopie (Perkin Elumer) wurde die Ca-Konzentration dieser Lösung bestimmt.

In der nachfolgenden Tabelle 1 sind erfindungsgemäße Zusammensetzungen als Konditioniermittel aufgeführt, die durch Verdünnen kommerziell erhältlicher Säuren und Komplexbildner in destilliertem Wasser mittels Magnetrührer hergestellt wurden. Die Zusammensetzungen 1 bis 3 sind Vergleichsbeispiele, die Zusammensetzungen 4 bis 7 erfindungsgemäße Beispiele. Die letzte Spalte der Tabelle 1 gibt die nach dem oben beschriebenen Versuch erhaltene Calciumkonzentration für die jeweilige Säure an. Alle Prozentangaben sind Gew.-%.

Tabelle 1:

| | Säure [%] | pKs (reine Säure) | Komplexbildner [%] | Ca-Konz. [mg/l] |
|---|---|---|---|---|
| 1. | Salzsäure 1% | -7 | - | 4,9 |

(fortgesetzt)

| | Säure [%] | pKs (reine Säure) | Komplexbildner [%] | Ca-Konz. [mg/l] |
|---|---|---|---|---|
| 2. | Salzsäure 5% | -7 | - | 17,7 |
| 3. | Salzsäure 15% | -7 | - | 20,35 |
| 4. | Citronensäure 60% | 3,1 | HEDP* 6% | 5,1 |
| 5. | Salzsäure 5% | -7 | HEDP 6% | 42,4 |
| 6. | Salzsäure 5% | -7 | HEDP 18% | 42,8 |
| 7. | p-Toluolsul-fonsäure 10% | -2 | HEDP 6% | 47,4 |
| *1-Hydroxyethan-1,1-diphosphonsäure | | | | |

Die Tabelle zeigt, dass die erfindungsgemäßen Zusammensetzungen selbst gegenüber 15%iger Salzsäure eine deutlich verbesserte Ätzwirkung aufweisen, ohne die beschriebenen Nachteile der Salzsäure aufzuweisen.

Aus den Zusammensetzungen 2, 3 und 5 werden Ätzgele hergestellt. Zu diesem Zweck wurden die Lösungen angedickt durch Zusatz von 5 Gew.-% pyrogener Kieselsäure (Aerosil® 200, Degussa) und 0,5 Gew.-% sorbitolbasiertes Polyetherpolyol mit einer Hydroxylzahl von 500. Die Vermischung erfolgt in einem Speedmixer (DAC 150 FV, Fa. Hauschild).

Aus gesunden kariesfreien humanen Molaren wurden Schmelzproben hergestellt. Die Schmelzoberfläche wurde mit Nagellack derart abgedeckt, dass unbedeckte Schmelzfenster entstanden. Anschließend wurden die zu testenden Ätzgele auf die Schmelzfenster appliziert. Nach 120 s wurden die Gele mit einem Wasserstrahl entfernt. Die Proben wurden getrocknet und in mit Fluoreszenzfarbstoff markierten (0,1 mM RITC) Spurr's Resin eingebettet. Die Untersuchung erfolgte mit einem Konfokalen Laserrastermikroskop (CLSM) im Fluoreszenzmodus. Eine nähere Beschreibung des Verfahrens findet sich in der Publikation Meyer-Lückel H. et al, Surface layer erosion of natural caries lesions wit phosphoric and hydrochloric acid gels in preparation for resin infiltration, caries research 2007, 41, 223-230.

| Gel | Einwirkzeit [s] | Erosionstiefe [$\mu$m] |
|---|---|---|
| 15% HCl | 120 | 27,3 |
| 5% HCl | 120 | 13,3 |
| 5% HCl + 18% HEDP | 120 | 25,6 |

Diese Beispiele und Vergleichsbeispiele zeigen, dass eine erfindungsgemäße Zusammensetzung eine hohe Erosionstiefe ermöglicht, ohne die beschriebenen Nachteile einer hochkonzentrierten Salzsäurelösung aufzuweisen. Die angegebenen Erosionstiefen sind Mittelwerte aus 8 Messungen.

Die bei den Ausführungsbeispielen verwendeten Säuren bilden unter den Bedingungen der Anwendung keine unlöslichen Calciumsalze. Dies ist generell ein bevorzugtes Merkmal der Erfindung.

**Patentansprüche**

1. Zusammensetzung, die enthält:

- 1 bis 80 Gew.-% wenigstens einer Säure, die einen pKs-Wert von 2 oder weniger aufweist;
- ein protisches Lösungsmittel;
- einen Komplexbildner für $Ca^{2+}$ - Ionen;

zur Verwendung als Konditioniermittel für das Ätzen von Schmelzläsionen.

2. Zusammensetzung nach Anspruch 1, **dadurch gekennzeichnet, dass** der Säuregehalt 1 bis 50 Gew.-%, vorzugsweise 4 bis 30 Gew.-%, weiter vorzugsweise 4 bis 15 Gew.-% beträgt.

3. Zusammensetzung nach Anspruch 1 oder 2, **dadurch gekennzeichnet, dass** der pKs-Wert der Säure -10 bis 1, vorzugsweise -10 bis 0 beträgt.

4. Zusammensetzung nach einem der Ansprüche 1 bis 3, **dadurch gekennzeichnet, dass** die Säure ausgewählt ist aus der Gruppe bestehend aus Perchlorsäure, Salzsäure, Schwefelsäure, Salpetersäure, Fluorsulfonsäure, Trifluormethansulfonsäure, Methansulfonsäure, Toluolsulfonsäure, Trichloressigsäure, Trifluoressigsäure, Pikrinsäure und Semiquadratsäure; weiter bevorzugt ausgewählt aus der Gruppe bestehend aus Salzsäure, Salpetersäure, Methansulfonsäure und Toluolsulfonsäure

5. Zusammensetzung nach einem der Ansprüche 1 bis 4, **dadurch gekennzeichnet, dass** der Komplexbildner mehrzähnig ist, vorzugsweise ein Chelatbildner ist.

6. Zusammensetzung nach Anspruch 5, **dadurch gekennzeichnet**, das der Chelatbildner ausgewählt ist aus der Gruppe bestehend aus Ethylendiamintetraessigsäure (EDTA), Diethylentriaminpentaessigsäure (DTPA), Nitrilotriessigsäure (NTA), Iminodiessigsäure (IDA), Glycolsäure, 4-Hydroxybutansäure (*Liquid Ecstasy*), Ethylendiamintetra(-methylenphosphonsäure) (DTPMP), Diethylentriaminpenta(-methylenphosphonsäure) (EDTMP), Aminotrismethylenphosphonsäure (ATMP), Hydroxyethylamino-di(methylenphosphonsäure) (HEMPA), Hexamethylendiamintetra(methylenphosphonsäure) (HDTMP), Phosphonobutan-1,2,4-tricarbonsäure (PBTC), und 1-Hydroxyethan-1,1-diphosphonsäure (HEDP).

7. Zusammensetzung nach einem der Ansprüche 1 bis 6, **dadurch gekennzeichnet, dass** der Komplexbildnergehalt 0,1 bis 80 Gew.-%, vorzugsweise 1 bis 80 Gew.-%, weiter vorzugsweise 5 bis 60 Gew.-%, weiter vorzugsweise 10 bis 40 Gew.-% beträgt.

8. Zusammensetzung nach einem der Ansprüche 1 bis 7, **dadurch gekennzeichnet, dass** sie zusätzlich Verdickungsmittel enthält, vorzugsweise Verdickungsmittel ausgewählt aus der Gruppe bestehend aus partikulären Füllstoffen und Polyethern.

9. Kit zur Durchführung einer Infiltration von Zahnschmelz, mit den Bestandteilen:

   - einem Konditionierer, der eine Zusammensetzung nach einem der Ansprüche 1 bis 7 aufweist;
   - einem Infiltranten.

10. Kit nach Anspruch 9, **dadurch gekennzeichnet, dass** der Infiltrant einen Penetrationskoeffizienten PK > 50 cm/s aufweist.

11. Kit nach Anspruch 10, **dadurch gekennzeichnet, dass** der Infiltrant wenigstens ein Harz enthält ausgewählt aus der Gruppe bestehend aus MMA, Methylmethacrylat; EMA, Ethylmethacrylat; n-BMA, n-Butylmethacrylat; IBMA, Isobutylmethacrylat, t-BMA, tert-Butylmethacrylat; EHMA, 2-Ethylhexylmethacrylat; LMA, Laurylmethacrylat; TDMA, Tridecylmethacrylat; SMA, Stearylmethacrylat; CHMA, Cyclohexylmethacrylat; BZMA, Benzylmethacrylat; IBXMA, Isobornylmethacrylate; MAA, Methacrylsäure; HEMA, 2-Hydroxyethylmethacrylat; HPMA, 2-Hydroxypropylmethacrylat; DMMA, Dimethylaminoethylmethacrylat; DEMA, Diethylaminoethylmethacrylat; GMA, Glycidylmethacrylat; THFMA, Tetrahydrofurfurylmethacrylat; AMA, Allylmethacrylat; EGDMA, Ethylenglycoldimethacrylat; 3EGDMA, Triethylenglycoldimethacrylat; 4EGDMA, Tetraethylenglycoldimethacrylat; BDMA, 1,3-Butylenglycoldimethacrylat; HDDMA, 1,6-Hexandioldimethacrylat; ETMA, Ethoxyethylmethacrylat; 3FM, Trifluorethylmethacrylat; 8FM, Octafluorpentylmethacrylat; AIB, Isobutylacrylat; TBA, tert-Butylacrylat; LA, Laurylacrylat; CEA, Cetylacrylat; STA, Stearylacrylat; CHA, Cyclohexylacrylat; BZA, Benzylacrylat; IBXA, Isobornylacrylat; 2-MTA, 2-Methoxyethylacrylat; ETA, 2-Ethoxyethylacrylat; EETA, Ethoxyethoxyethylacrylat; PEA, 2-Phenoxyethylacrylat; THFA, Tetrahydrofurylacrylat; HEA, 2-Hydroxyethylacrylat; HPA, 2-Hydroxypropylacrylat; 4HBA, 4-Hydroxybutylacrylat; DMA, Dimethylaminoethylacrylat; 1,4-Butylendioldiacrylat; 4EDA, Tetraethylenglycoldiacrylat; NDDA, 1,9- Nonandioldiacrylat; 3F, Trifluorethylacrylat; 17F, Heptadecafluorodecylacrylat; 2-PEA, 2-Phenoxyethylacrylat; TBCH, 4-tert-butylcyclohexylacrylat; DCPA, Dihydrodicyclopentadienylacrylat; EHA, 2-Ethylhexylacrylat; 3EGMA, Triethylenglycolmonomethacrylat; DEGDMA, Diethylenglycoldimethacrylat; PDDMA, 1,5-Pentandioldimethacrylat; BDDMA, 1,4-Butandioldimethacrylat; PRDMA, 1,3-Propandioldimethacrylat; DDDMA, 1,10-Decandioldimethacrylat; PEG400DA, Polyethylenglycol 400 Diacrylat, TMPTMA, Trimethylolpropantrimethacrylat, TMPTA, Trimethylolpropantriacrylat; DTMPTA; Di-Trimethylolpropantetraacrylat; DiPENTA, Di-Pentaerythritolpentaacrylat; PEG400DMA, Polyethylenglycol 400 Dimethacrylat, PEG300DA, Polyethylenglycol 300 Diacrylat, PEG300DMA, Polyethylenglycol 300 Dimethacrylat, BPA(EO)10DMA, Ethoxyliertes (10) Bisphenol-A-Dimethacrylat; BPA(EO)30DMA, Ethoxyliertes (30) Bisphenol-A-Dimethacrylat; PEG200DA, Polyethylenglycol 200 Diacrylat, PEG600DA, Polyethylenglycol 600 Diacrylat; NPG(PO)2DA Propoxyliertes (2) Neopentylglycol Diacrylat; BPA(EO)2DA, Ethoxyliertes (4) Bisphenol-A-Diacrylat; GPTA; propoxyliertes Glyceryltriacrylat; DMTCDDA, Dimethylol tricyclo[5.2.1.0]decandimethacrylat; BPA(PO)2DMA, Propoxyliertes (2); Bisphenol-A-Dimethacrylat; DPEHA, Dipentaerythritolhexaacrylat; Bis-GMA, 2,2-bis [4-(2-Hydroxy-3-Methacryloxypropoxy)phenyl]propan; und UDMA, 1,6-bis(Methacryloxy-2-Ethoxycarbonylamino)-2,4,4-trimethylhexan.

12. Kit nach Anspruch 10 oder 11, **dadurch gekennzeichnet, dass** der Infiltrant wenigstens ein Harz enthält ausgewählt aus der Gruppe bestehend aus Polymethacrylsäuren und deren Derivaten; vorzugsweise ausgewählt aus der Gruppe bestehend aus TEGDMA, Triethylenglycoldimethacrylat; und TMPTMA, Trimethylolpropantrimethacrylat.

13. Kit nach einem der Ansprüche 10 bis 12, **dadurch gekennzeichnet, dass** er wenigstens einen Applikationsstreifen und/oder Reinigungsstreifen und/oder eine Trenneinrichtung enthält.

14. Kit nach Anspruch 13, **dadurch gekennzeichnet, dass** er einen mit einem Konditionierer versehenen Applikationsstreifen enthält.

15. Kit nach Anspruch 13 oder 14, **dadurch gekennzeichnet, dass** er einen mit einem Infiltranten versehenen Applikationsstreifen enthält.

**Claims**

1. Composition comprising:

   - 1 to 80 % by weight of at least one acid which has a pKa of 2 or less;
   - a protic solvent;
   - a complexing agent for $Ca^{2+}$ ions;
   for use as conditioning agent for etching enamel lesions.

2. Composition according to claim 1, **characterised in that** the acid content is from 1 to 50 % by weight, preferably 4 to 30 % by weight, more preferably 4 to 15 % by weight.

3. Composition according to either claim 1 or claim 2, **characterised in that** the pKa of the acid is from -10 to 1, preferably -10 to 0.

4. Composition according to any of claims 1 to 3, **characterised in that** the acid is selected from the group consisting of perchloric acid, hydrochloric acid, sulfuric acid, nitric acid, fluorosulfonic acid, trifluoromethanesulfonic acid, methanesulfonic acid, toluenesulfonic acid, trichloroacetic acid, trifluoroacetic acid, picric acid and semisquaric acid; more preferably selected from the group consisting of hydrochloric acid, nitric acid, methanesulfonic acid and toluenesulfonic acid.

5. Composition according to any of claims 1 to 4, **characterised in that** the complexing agent is multidentate, and is preferably a chelating agent.

6. Composition according to claim 5, **characterised in that** the chelating agent is selected from the group consisting of ethylenediaminetetraacetic acid (EDTA), diethylenetriaminepentaacetic acid (DTPA), nitrilotriacetic acid (NTA), iminodiacetic acid (IDA), glycolic acid, 4-hydroxybutanoic acid (liquid ecstasy), ethylenediaminetetra(methylenephosphonic acid) (DTPMP), diethylenetriaminepenta(methylenephosphonic acid) (EDTMP), aminotrismethylenephosphonic acid (ATMP), hydroxyethylaminodi(methylenephosphonic acid) (HEMPA), hexamethylene-diaminetetra(methylenephosphonic acid) (HDTMP), phosphonobutane-1,2,4-tricarboxylic acid (PBTC), and 1-hydroxyethane-1,1-diphosphonic acid (HEDP).

7. Composition according to any of claims 1 to 6, **characterised in that** the complexing agent content is from 0.1 to 80 % by weight, preferably 1 to 80 % by weight, more preferably 5 to 60 % by weight, more preferably 10 to 40 % by weight.

8. Composition according to any of claims 1 to 7, **characterised in that** said composition additionally comprises thickeners, preferably thickeners selected from the group consisting of particulate fillers and polyethers.

9. Kit for carrying out an infiltration of dental enamel, comprising the constituents:

- a conditioner which has a composition according to any of claims 1 to 7;
- an infiltrant.

10. Kit according to claim 9, **characterised in that** the infiltrant has a penetration coefficient PC of > 50 cm/s.

11. Kit according to claim 10, **characterised in that** the infiltrant comprises at least one resin selected from the group consisting of MMA, methyl methacrylate; EMA, ethyl methacrylate; n-BMA, n-butyl methacrylate; IBMA, isobutyl methacrylate, t-BMA, tert-butyl methacrylate; EHMA, 2-ethylhexyl methacrylate; LMA, lauryl methacrylate; TDMA, tridecyl methacrylate; SMA, stearyl methacrylate; CHMA, cyclohexyl methacrylate; BZMA, benzyl methacrylate; IBXMA, isobornyl methacrylate; MAA, methacrylic acid; HEMA, 2-hydroxyethyl methacrylate; HPMA, 2-hydroxypropyl methacrylate; DMMA, dimethylaminoethyl methacrylate; DEMA, diethylaminoethyl methacrylate; GMA, glycidyl methacrylate; THFMA, tetrahydrofurfuryl methacrylate; AMA, allyl methacrylate; EGDMA, ethylene glycol dimethacrylate; 3EGDMA, triethylene glycol dimethacrylate; 4EGDMA, tetraethylene glycol dimethacrylate; BDMA, 1,3-butylene glycol dimethacrylate; HDDMA, 1,6-hexanediol dimethacrylate; ETMA, ethoxyethyl methacrylate; 3FM, trifluoroethyl methacrylate; 8FM, octafluoropentyl methacrylate; AIB, isobutyl acrylate; TBA, tert-butyl acrylate; LA, lauryl acrylate; CEA, cetyl acrylate; STA, stearyl acrylate; CHA, cyclohexyl acrylate; BZA, benzyl acrylate; IBXA, isobornyl acrylate; 2-MTA, 2-methoxyethyl acrylate; ETA, 2-ethoxyethyl acrylate; EETA, ethoxyethoxyethyl acrylate; PEA, 2-phenoxyethyl acrylate; THFA, tetrahydrofurfuryl acrylate; HEA, 2-hydroxyethyl acrylate; HPA, 2-hydroxypropyl acrylate; 4HBA, 4-hydroxybutyl acrylate; DMA, dimethylaminoethyl acrylate; 1,4-butylenediol diacrylate; 4EDA, tetraethylene glycol diacrylate; NDDA, 1,9-nonanediol diacrylate; 3F, trifluoroethyl acrylate; 17F, heptadecafluorodecyl acrylate; 2-PEA, 2-phenoxyethyl acrylate; TBCH, 4-tert-butylcyclohexyl acrylate; DCPA, dihydrodicyclopentadienyl acrylate; EHA, 2-ethylhexyl acrylate; 3EGMA, triethylene glycol monomethacrylate; DEGDMA, diethylene glycol dimethacrylate; PDDMA, 1,5-pentanediol dimethacrylate; BDDMA, 1,4-butanediol dimethacrylate; PRDMA, 1,3-propanediol dimethacrylate; DDDMA, 1,10-decanediol dimethacrylate; PEG400DA, polyethylene glycol 400 diacrylate, TMPTMA, trimethylolpropane trimethacrylate, TMPTA, trimethylolpropane triacrylate; DTMPTA; ditrimethylolpropane tetraacrylate; DiPENTA, di-pentaerythritol pentaacrylate; PEG400DMA, polyethylene glycol 400 dimethacrylate, PEG300DA, polyethylene glycol 300 diacrylate, PEG300DMA, polyethylene glycol 300 dimethacrylate, BPA(EO)10DMA, ethoxylated (10) bisphenol A dimethacrylate; BPA(EO)30DMA, ethoxylated (30) bisphenol A dimethacrylate; PEG200DA, polyethylene glycol 200 diacrylate, PEG600DA, polyethylene glycol 600 diacrylate; NPG(PO)2DA propoxylated (2) neopentyl glycol diacrylate; BPA(EO)2DA, ethoxylated (4) bisphenol A diacrylate; GPTA; propoxylated glyceryl triacrylate; DMTCDDA, dimethylol tricyclo[5.2.1.0$^{2,6}$]decane dimethacrylate; BPA(PO)2DMA, propoxylated (2) bisphenol A dimethacrylate; DPEHA, dipentaerythritol hexaacrylate; bis-GMA, 2,2-bis[4-(2-hydroxy-3-methacryloxypropoxy)phenyl]propane; and UDMA, 1,6-bis(methacryloxy-2-ethoxycarbonylamino)-2,4,4-trimethylhexane.

12. Kit according to either claim 10 or claim 11, **characterised in that** the infiltrant comprises at least one resin selected from the group consisting of polymethacrylic acids and derivatives thereof; preferably selected from the group consisting of TEGDMA, triethylene glycol dimethacrylate; and TMPTMA, trimethylolpropane trimethacrylate.

13. Kit according to any of claims 10 to 12, **characterised in that** said kit comprises at least one application strip and/or cleaning strip and/or a separator.

14. Kit according to claim 13, **characterised in that** said kit comprises an application strip provided with a conditioner.

15. Kit according to either claim 13 or claim 14, **characterised in that** said kit comprises an application strip provided with an infiltrant.

## Revendications

1. Composition contenant :

    - 1 à 80 % en poids d'au moins un acide qui présente un pKs de 2 ou moins ;
    - un solvant protique ;
    - un complexant des ions Ca$^{2+}$ ;

    pour utilisation en tant qu'agent de conditionnement pour le décapage des lésions de l'émail.

**2.** Composition selon la revendication 1, **caractérisée en ce que** la teneur en acide est de 1 à 50 % en poids, de préférence de 4 à 30 % en poids, d'une manière plus préférée de 4 à 15 % en poids.

**3.** Composition selon la revendication 1 ou 2, **caractérisée en ce que** le pKs de l'acide est de -10 à 1, de préférence de -10 à 0.

**4.** Composition selon l'une des revendications 1 à 3, **caractérisée en ce que** l'acide est choisi dans le groupe consistant en l'acide perchlorique, l'acide chlorhydrique, l'acide sulfurique, l'acide nitrique, l'acide fluorosulfonique, l'acide trifluorométhanesulfonique, l'acide méthanesulfonique, l'acide toluènesulfonique, l'acide trichloracétique, l'acide trifluoracétique, l'acide picrique et l'acide semiquadratique ; et d'une manière plus préférée est choisi dans le groupe consistant en l'acide chlorhydrique, l'acide nitrique, l'acide méthanesulfonique et l'acide toluènesulfonique.

**5.** Composition selon l'une des revendications 1 à 4, **caractérisée en ce que** l'agent complexant est multidenté, et est de préférence un chélatant.

**6.** Composition selon la revendication 5, **caractérisée en ce que** le chélatant est choisi dans le groupe consistant en l'acide éthylènediaminetétraacétique (EDTA), l'acide diéthylènetriaminepentaacétique (DTPA), l'acide nitrilotriacétique (NTA), l'acide iminodiacétique (IDA), l'acide glycolique, l'acide 4-hydroxybutanoïque (*Liquid Ecstasy*), l'acide éthylènediaminetétra(méthylènephosphonique) (DTPMP), l'acide diéthylènetriaminepenta(méthylènephosphonique) (EDTMP), l'acide aminotrisméthylènephosphonique (ATMP), l'acide hydroxyéthylamino-di(méthylènephosphonique) (HEMPA), l'acide hexaméthylènediamine-tétra(méthylènephosphonique) (HDTMP), l'acide phosphonobutane-1,2,4-tricarboxylique (PBTC) et l'acide 1-hydroxyéthane-1,1-diphosphonique (HEDP).

**7.** Composition selon l'une des revendications 1 à 6, **caractérisée en ce que** la teneur en l'agent complexant est de 0,1 à 80 % en poids, de préférence de 1 à 80 % en poids, d'une manière plus préférée de 5 à 60 % en poids, d'une manière plus préférée de 10 à 40 % en poids.

**8.** Composition selon l'une des revendications 1 à 7, **caractérisée en ce qu'**elle contient en outre des épaississants, de préférence des épaississants choisis dans le groupe consistant en les matières de charge particulaires et les polyéthers.

**9.** Trousse pour la mise en oeuvre d'une infiltration d'émail dentaire, contenant les constituants suivantes :

- un agent de conditionnement qui a une composition selon l'une des revendications 1 à 7 ;
- un infiltrant.

**10.** Trousse selon la revendication 9, **caractérisée en ce que** l'infiltrant présente un coefficient de pénétration PK > 50 cm/s.

**11.** Trousse selon la revendication 10, **caractérisée en ce que** l'infiltrant contient au moins une résine choisie dans le groupe consistant en le MMA, méthacrylate de méthyle ; l'EMA, méthacrylate d'éthyle ; le n-BMA, méthacrylate de n-butyle ; l'IBMA, méthacrylate d'isobutyle ; le t-BMA, méthacrylate de tert-butyle ; l'EHMA, méthacrylate de 2-éthylhexyle ; le LMA, méthacrylate de lauryle ; le TDMA, méthacrylate de tridécyle ; le SMA, méthacrylate de stéaryle ; le CHMA, méthacrylate de cyclohexyle ; le BZMA, méthacrylate de benzyle ; l'IBXMA, méthacrylate d'isobornyle ; le MAA, acide méthacrylique ; le HEMA, méthacrylate de 2-hydroxyéthyle ; le HPMA, méthacrylate de 2-hydroxypropyle ; le DMMA, méthacrylate de diméthylaminoéthyle ; le DEMA, méthacrylate de diéthylaminoéthyle ; le GMA, méthacrylate de glycidyle ; le THFMA, méthacrylate de tétrahydrofurfuryle ; l'AMA, méthacrylate d'allyle ; l'EGDMA, diméthacrylate d'éthylèneglycol ; le 3EGDMA, diméthacrylate de triéthylèneglycol ; le 4EGDMA, diméthacrylate de tétraéthylèneglycol ; le BDMA, diméthacrylate de 1,3-butylèneglycol ; le HDDMA, diméthacrylate de 1,6-hexanediol ; l'ETMA, méthacrylate d'éthoxyéthyle ; le 3FM, méthacrylate de trifluoréthyle ; le 8FM, méthacrylate d'octafluoropentyle ; l'AIB, acrylate d'isobutyle ; le TBA, acrylate de tert-butyle ; le LA, acrylate de lauryle ; le CEA, acrylate de cétyle ; le STA, acrylate de stéaryle ; le CHA, acrylate de cyclohexyle, le BZA, acrylate de benzyle ; l'IBXA, acrylate d'isobornyle ; le 2-MTA, acrylate de 2-méthoxyéthyle ; l'ETA, acrylate de 2-éthoxyéthyle ; l'EETA, acrylate d'éthoxyéthoxyéthyle ; le PEA, acrylate de 2-phénoxyéthyle ; le THFA, acrylate de tétrahydrofurfuryle ; le HEA, acrylate de 2-hydroxyéthyle ; le HPA, acrylate de 2-hydroxypropyle ; le 4HBA, acrylate de 4-hydroxybutyle ; le DMA, acrylate de diméthylaminoéthyle ; le diacrylate de 1,4-butylènediol ; le 4EDA, diacrylate de tétraéthylèneglycol, le NDDA, diacrylate de 1,9-nonanediol ; le 3F, acrylate de trifluoréthyle ; le 17F, acrylate d'heptadécafluorodécyle ; le 2-PEA, acrylate de 2-phénoxyéthyle ; le TBCH, acrylate de 4-tert-butylcyclohexyle ; le DCPA, acrylate de dihydrodicyclopentadiényle ; l'EHA, acrylate de 2-éthylhexyle ; le 3EGMA, monométhacrylate de triéthylèneglycol ; le DEGDMA, diméthacrylate de diéthylèneglycol ; le PDDMA, diméthacrylate de 1,5-pentanediol ; le BDDMA, diméthacrylate de 1,4-butanediol ; le PRDMA, diméthacrylate de 1,3-propanediol ; le DDDMA, diméthacrylate de 1,10-décanediol ; le PEG400DA, diacrylate de polyéthylèneglycol 400 ; le TMPTMA, triméthacrylate de triméthylolpropane ; le TMPTA, triacrylate de triméthylolpropane ; le DTMPTA, tétraacrylate de di-triméthylolpropane ; le DiPENTA, pentaacrylate de dipentaérythritol ; le PEG400DMA, diméthacrylate de polyéthylèneglycol 400 ; le PEG300DA, diacrylate de polyéthylèneglycol 300 ; le PEG300DMA, diméthacrylate de polyéthylèneglycol 300 ; le BPA(EO)100DMA, diméthacrylate de bisphénol A éthoxylé (10) ; le BPA(EO)30DMA, diméthacrylate de bisphénol A éthoxylé (30) ; le PEG200DA, diacrylate de polyéthylèneglycol 200 ; le PEG600DA, diacrylate de polyéthylèneglycol 600 ; le NPG(PO)2DA, diacrylate de néopentylglycol propoxylé (2) ; le BPA(EO)2DA, diacrylate de bisphénol-A éthoxylé (4) ; le GPTA, triacrylate de glycéryle propoxylé ; le DMTCDDA, diméthacrylate de diméthylol-tricyclo[5.2.1.0]décane ; le BPA(PO)2DMA, diméthacrylate de bisphénol A propoxylé (2) ; le DPEHA, hexaacrylate de dipentaérythritol ; le Bis-GMA, 2,2-bis[4-(2-hydroxy-3-méthacryloxy-propoxy)phényl]propane ; et l'UDMA, 1,6-bis(méthacryloxy-2-éthoxycarbonylamino)-2,4,4-triméthylhexane.

12. Trousse selon la revendication 10 ou 11, **caractérisée en ce que** l'infiltrant contient au moins une résine choisie dans le groupe consistant en les acides polyméthacryliques et leurs dérivés ; de préférence choisie dans le groupe consistant en le TEGDMA, diméthacrylate de triéthylèneglycol ; et le TMPTMA, triméthacrylate de triméthylolpropane.

13. Trousse selon l'une des revendications 10 à 12, **caractérisée en ce qu'**elle contient au moins une bande d'application et/ou une bande de nettoyage et/ou un système de séparation.

14. Trousse selon la revendication 13, **caractérisée en ce qu'**elle contient une bande d'application pourvue d'un agent de conditionnement.

15. Trousse selon la revendication 13 ou 14, **caractérisée en ce qu'**elle contient une bande d'application pourvue d'un infiltrant.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007131725 A1

**In der Beschreibung aufgeführte Nicht-Patentliteratur**

- **A. KLOCKE et al.** *Dental Materials,* 2003, vol. 19, 773

- **MEYER-LÜCKEL H. et al.** Surface layer erosion of natural caries lesions wit phosphoric and hydrochloric acid gels in preparation for resin infiltration. *caries research,* 2007, vol. 41, 223-230